# EUROPEAN PATENT APPLICATION

(11) **EP 2 177 221 A1**
(43) Date of publication of application: **21.04.2010**
(21) Application number: 08167069.7
(22) Date of filing: 20.10.2008
(51) Int. Cl.: A61K 31/4453, A61P 5/50, C07D 295/135

(54) **Process for the preparation of substantially optically pure Repaglinide and precursors thereof**

(71) Applicant: Krka Tovarna Zdravil, D.D., Novo Mesto, 8501 Novo Mesto (SI)
(72) Inventor: Veverka, Miroslav, 847 08 Bratislava (SK); Veverkova, Eva, 847 08 Bratislava (SK); Klvanova, Jana, 82107 Bratislava (SK); Bombek, Sergeja, 8000 Novo mesto (SI); Marjo, Merslavic, 8351 Straza (SI); Smodis, Janez, 8000 Novo mesto (SI)
(74) Representative: UEXKÜLL & STOLBERG

(57) **Abstract**

The invention relates to a process for preparing substantially optically pure Repaglinide and pharmaceutically acceptable salts, solvates and esters thereof, as well as precursors therefore.

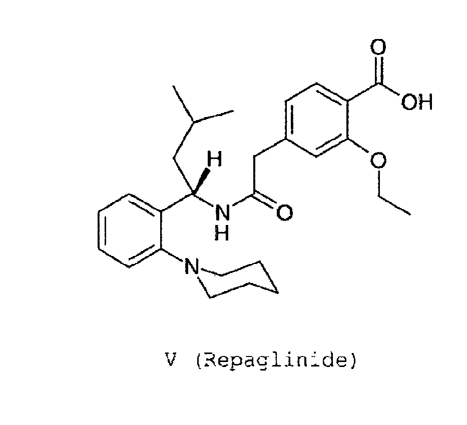

## Description

### Technical field

The invention relates to a process for preparing substantially optically pure Repaglinide and pharmaceutically acceptable salts, solvates and esters thereof. The invention further relates to a process for preparing precursors of repaglinide and in particular for preparing (S)(+)-1-(2-piperidino-phenyl)-3-methyl-1-butylamine.

### Background of the invention

Repaglinide is the generic name for (S)-2-ethoxy-4-[N-[1-(2-piperidinophenyl)-3-methyl-1-butyl]aminocarbonylmethyl]-benzoic acid of the following formula V 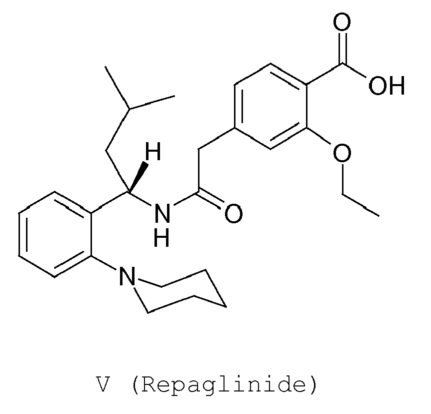 which is the pharmacologically active enantiomer.

Repaglinide is a known non-sulfonylurea hypoglycemic agent, which has an insulinotropic effect by blocking the K_{ATP}-channels of pancreatic B-cells. It is particularly used for lowering blood glucose levels in patients suffering from type II diabetes mellitus (NIDDM). The drug is currently being marketed in oral dosage forms containing 0.5mg, 1mg and 2mg Repaglinide.

Repaglinide and related compounds, as well as the preparation thereof, are disclosed in EP 0 147 850, EP 0 207 331, EP 0 589 874, EP 0 965 591, WO 2004/101540 and Grell et al. in J. Med. Chem. Vol. 41(26), p. 5219-5246, 1998. Crystal forms of Repaglinide are known from EP 589 874, WO 2004/018442, WO 2005/021524 and WO 2004/101540. In the following description, any reference to the term "Repaglinide" is intended to include its (S)-enantiomer, solvates, esters and salts thereof in any amorphous or crystalline form.

In general, the processes for the preparation of Repaglinide as are known from the prior art comprise the coupling of (S)(+)-1-(2-piperidino-phenyl)-3-methyl-1-butylamine with a substituted carboxylic acid to obtain Repaglinide ester. Optically pure Repaglinide is finally obtained by converting the ester-protected carboxyl group to the free carboxyl group, followed by column chromatography to improve yield and purity of the product.

The coupling step of these processes is usually performed by using a solvent selected from a large group of organic solvents together with acid-activating or dehydrating agents, or agents which activate the amino group.

EP 0 589 874 discloses a process for the preparation of repaglinide ethyl ester, wherein the coupling step is preferably performed using N,N'-dicyclohexyl carbodiimide as an acid-activating compound in toluene, or using triphenylphosphine and triethylamine as dehydrating agents in CCl₄. After recrystallisation the ester product was obtained in a yield of 74.3% and 77.3%, respectively.

EP 0 147 850 refers to a coupling reaction of (S)(+)-1-(2-piperidino-phenyl)-1-butylamine with 2-(3-ethoxy-4-(ethoxycarbonyl)phenyl)acetic acid wherein N,N'-Carbonyldiimidazole is used as an acid-activating compound in THF. After purification by column chromatography the yield of the ester product was 51.2%.

WO 2004/101540 discloses a process for the preparation of repaglinide comprising coupling of an amine compound with 2-(3-ethoxy-4-(ethoxycarbonyl)phenyl)acetic acid or its derivative. The acid-activating compound preferably used therein is 1,1'-carbonyldiimidazol (CDI) in a mixture of toluene and acetonitrile.

Grell et al. in J. Med. Chem. Vol. 41(26), p. 5219-5246, 1998 refer to a synthesis of benzoic acid derivatives, wherein the above mentioned coupling reaction is performed using CDI in THF, triphenylphosphine/CCl₄/triethylamine in acetonitrile, or N,N'-dicyclohexyl carbodiimide in toluene. To afford the targeted ester in a satisfactory high yield and purity, the crude product was purified by column chromatography.

A general problem of these conventional processes is to obtain the repaglinide ester in a satisfactory yield and purity.

The above prior art documents also disclose a process for the preparation of the optically pure (S)(+)-1-(2-piperidino-phenyl)-3-methyl-1-butylamine educt. In that process, the racemic 1-(2-piperidino-phenyl)-3-methyl-1-butylamine is contacted with an optically active acid in order to obtain a mixture of diastereomeric addition salts, which can be separated and converted into the desired enantiomers. The optically active acid is selected from mandelic acid, substituted mandelic acid, camphor-sulfonic acid, tartaric acid, substituted tartaric acid, or optically active phosphoric acid.

EP 0 589 874 discloses a process for the preparation of (S)(+)-1-(2-piperidino-phenyl)-3-methyl-1-butylamine by salt formation of the corresponding racemic amine with N-acetyl glutamic acid in a solvent mixture comprising methanol and acetone. After precipitation the addition salt has to be recrystallized to obtain the (S)-enantiomer in a satisfactory yield and purity.

Grell et al. in J. Med. Chem. Vol. 41(26), p. 5219-5246, 1998 describe an analogous process for the preparation of (S)(+)-1-(2-piperidino-phenyl)-3-methyl-1-butylamine. Also in this process, recrystallization of the crude precipitate is necessary to obtain an optical purity of 98% (e.e.).

WO 2004/101540 discloses the use of L-mandelic acid as optically active acid together with ethyl acetate or acetonitrile as a solvent. After filtration the product has to be recrystallized at least once to yield the desired (S)-amine in an optical purity of >95% (e.e.).

Thus, the problem of the prior art processes for the preparation of substantially optically (S)(+)-1-(2-piperidino-phenyl)-3-methyl-1-butylamine is the necessity to perform repeated crystallization steps in order to achieve a satisfactory optical purity.

### Description of the invention

It is, therefore, an object of the present invention to provide a process for preparing substantially optically pure repaglinide which is economical and provides the product in a high yield and high optical purity. Therefore, a particular object is to provide a highly efficient process for preparing substantially optically pure (S) (+)-1-(2-piperidino-phenyl)-3-methyl-1-butylamine, which is a precursor used to prepare repaglinide. In particular, it is an object to improve the yield and optical purity of this precursor and at the same time to avoid time-consuming recrystallization and purification steps.

Thus, in a first aspect the invention relates to a process for the preparation of substantially optically pure (S)(+)-1-(2-piperidino-phenyl)-3-methyl-1-butylamine of the formula I 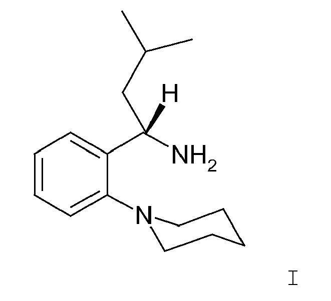 comprising:
a) contacting racemic 1-(2-piperidino-phenyl)-3-methyl-1 butylamine of the formula II 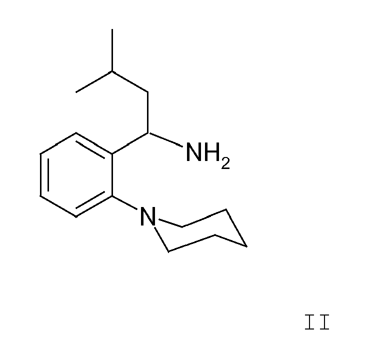 with an optically active acid in a solvent or solvent mixture to give a mixture of diastereomeric acid addition salts, wherein the optically active acid is O,O'-dibenzoyl-(+)-tartaric acid,
b) separating the mixture of diastereomeric acid addition salts to obtain substantially optically pure (S) (+)-1-(2-piperidino-phenyl)-3-methyl-1-butylamine O,O'-dibenzoyl-(+)-tartrate and
c) converting the (S) (+)-1-(2-piperidino-phenyl)-3-methyl-1-butylamine O,O'-dibenzoyl-(+)-tartrate obtained in step b) to substantially optically pure (S)(+)-1-(2-piperidino-phenyl)-3-methyl-1-butylamine of formula I.

In the following description and claims, (S)(+)-1-(2-piperidino-phenyl)-3-methyl-1-butylamine of formula I is also referred to as "(S)-amine" and (R) (-)-1-(2-piperidino-phenyl)-3-methyl-1-butylamine of formula VIII is also referred to as "(R)-amine".

The substantially optically pure (S)-amine obtained by the process according to the invention preferably has an optical purity of at least 70% (e.e.), more preferably at least 90% (e.e.) and most preferably at least 95% (e.e.), such as 100% (e.e.).

In a preferred embodiment, the contacting in step a) is performed at an elevated temperature, i.e. at about 50°C to the reflux temperature of the solvent. It is even more preferred to perform the contacting at about 60°C to about 90°C, such as about 85°C.

The optically active acid used in the present invention is O,O'-dibenzoyl-(+)-tartaric acid, also referred to as (+)-dibenzoyl tartaric acid. It is preferably used in an amount of from 0.25 to 0.75 molar equivalents and in particular in an amount of 0.5 molar equivalents, with respect to the racemic amine of formula II.

Suitable solvents for contacting the racemic amine of formula II and (+)-dibenzoyl tartaric acid to obtain the corresponding mixture of diastereomeric addition salts are selected from isobutylmethyl ketone, 1-butanole, methylethyl ketone, butyl acetate, 2-methylpropyl acetate, 2-butanone, 2-pentanone, toluene, 2-methylethyl acetate or mixtures thereof. Preferably, the reaction is performed in isobutylmethyl ketone and even more preferably in butyl acetate.

The separation of the mixture of the diastereomeric acid addition salts in step b) is typically performed by crystallization of the reaction mixture and isolation of the crystallized (S)-amine acid addition salt. In particular, the crystallization is performed by cooling the mixture to a temperature of about -20°C to about 20°C. More preferably, the mixture is cooled to a temperature of about -20°C to 10°C and most preferably to a temperature of about -20°C to -10°C. Finally, crystals are separated by filtration, optionally washed with a solvent, such as ethyl acetate or diethyl ether, and dried. In order to assess the efficiency of the process, the product is characterized by chiral HPLC.

Prior to the separation in step b) seeding crystals of (S)(+)-1-(2-piperidino-phenyl)-3-methyl-1-butylamine O,O'-dibenzoyl-(+)-tartrate can be added to the crystallization mixture. This seeding is preferably performed at a temperature of 0°C to the boiling point of the solvent, more preferably at a temperature of about 30°C to about 60°C, and most preferably at a temperature of about 40°C to about 50°C.

In step (c), the (S)-amine acid addition salt obtained in step (b) is converted to the free (S)-amine by e.g. adjusting the pH of a solution of the (S)-amine acid addition salt to about 7.5 to 10.

The process according to the present invention, which comprises the use of a specific optically active acid, i.e. (+)-dibenzoyl tartaric acid, has a number of advantages. The process in particular allows to efficiently provide the (S)-amine in an optical purity of >99 % (e.e.) by a single precipitation step of the corresponding (S)-amine acid addition salt and further conversion to the free amine, without the need for further crystallization or purification steps.

In another embodiment of step a) of the process according to the first aspect of the invention, instead of racemic amine of formula II a mixture comprising the (S)-amine and the (R)-amine in any molar ratio is contacted with O,O'-dibenzoyl-(+)-tartaric acid in a solvent or solvent mixture to give a mixture of diastereomeric acid addition salts. Such a mixture comprising the (S)-amine and the (R)-amine can be a mixture in which the amount of (S)-amine is higher than that of (R)-amine. For example, such a mixture is obtained by contacting racemic amine of formula II with O,O'-dibenzoyl-(-)-tartaric acid to give a mixture of diastereomeric acid addition salts, crystallizing and separating the (R)-amine acid addition salt to give an (S)-amine enriched mother liquor and neutralizing the obtained mother liquor to obtain a free (S)-amine enriched solution which can be used as starting material in a process according to the first aspect of the invention.

In a second aspect, the invention relates to a process for the preparation of a mixture comprising the (S)-amine of formula I 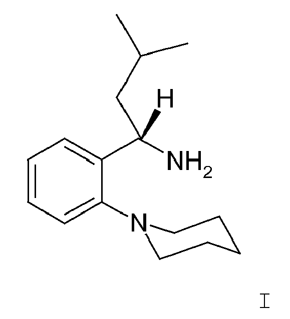 and the (R)-amine of formula VIII 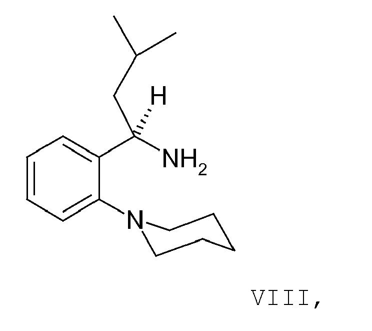 comprising treating a solution containing the (R)-amine with a racemization agent selected from the group consisting of ion exchange resins, potassium tert.-butoxide (t-BuOK) and mixtures thereof, wherein the amount of the (S)-amine in the obtained mixture is higher than in the solution containing the (R)-amine.

Preferably, the obtained mixture comprises the (S)-amine and the (R)-amine in a molar ratio of 40:60 to 60:40, more preferably of about 50:50.

Suitable examples of ion exchange resins which are useful as racemization agent include weakly acidic cation exchange resins, preferably macroporous methacrylic acid divinylbenzene copolymers such as Lewatit like Lewatit CNP 105.

Preferably, the reaction is performed at a temperature ranging from room temperature to the reflux temperature of the solvent used such as 25 to 30°C or 60 to 65°C.

The solvent can be selected from the group consisting of dimethylacetamide, dimethylformamide, dimethylsulfoxide, N-methylpyrrolidin-2-one, pyrrolidin-2-one, 1,4-dioxane, aliphatic alkanols such as methanol and ethanol, cyclic alkanols, ethylene glycol, diethylene glycol, diglyme and mixtures thereof.

Suitably, the starting (R)-amine containing solution which is treated according to the above process can be recovered from a fractional crystallization of diastereomeric salts.

In a preferred embodiment, an (R)-amine enriched mother liquor obtained from fractional crystallization using an optically active acid, such as a process described in the first aspect of the present invention, is evaporated to dryness to give crude (R)-enriched 1-(2-piperidino-phenyl)-3-methyl-1-butylamine acid addition salt. Then, crude (R)-enriched 1-(2-piperidino-phenyl)-3-methyl-1-butylamine is regenerated, e.g. by preparing the free amine base, extracting this base with an organic solvent and evaporating the solvent. Preferably, the organic solvent used in this extraction step is CH₂Cl₂. Further, ammonia is preferably used to free the amine base. Thereafter, a dry extract of (R)-enriched 1-(2-piperidino-phenyl)-3-methyl-1-butylamine is dissolved in an organic solvent and racemization is performed using a racemization agent at elevated temperature.

In a preferred embodiment, the organic solvent used in the racemization step is methanol and macroporous methacrylic acid divinylbenzene copolymer (Lewatit CNP 105) is used as racemization agent. This reaction is preferably performed at a temperature of about 25-30°C.

Moreover, it is preferred that the regenerated (R)-enriched 1-(2-piperidino-phenyl)-3-methyl-1-butylamine is dried azeotropically before racemization, preferably in toluene.

In a third aspect, the invention relates to a process for the preparation of substantially optically pure (S)-amine of the formula I 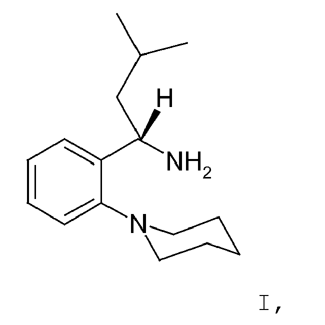 comprising:
a) treating a solution containing the (R)-amine of formula VIII 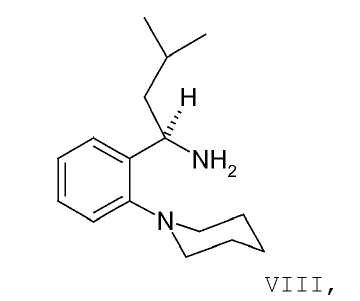 with a racemization agent to obtain a mixture comprising the (S)-amine of formula I and the (R)-amine of formula VIII, wherein the amount of the (S)-amine in the obtained mixture is higher than in the solution containing the (R)-amine;
b) contacting the mixture obtained in step a) with O,O'-dibenzoyl-(+)-tartaric acid in a solvent or solvent mixture to give a mixture of diastereomeric acid addition salts;
c) separating the mixture of diastereomeric acid addition salts to obtain substantially optically pure (S) (+)-1-(2-piperidino-phenyl)-3-methyl-1-butylamine O,O'-dibenzoyl-(+)-tartrate and
d) converting the (S) (+) -1- (2-piperidino-phenyl) -3-methyl-1-butylamine O,O'-dibenzoyl-(+)-tartrate obtained in step c) to substantially optically pure (S)(+)-1-(2-piperidino-phenyl)-3-methyl-1-butylamine of formula I.

Generally, the starting solution containing the (R)-amine comprises the (R)-amine in a higher amount than the (S)-amine. Moreover, the starting solution containing the (R)-amine can generally be obtained by any suitable enantiomer separation procedures such as chiral column chromatography, stereoselective synthesis or fractional crystallization. For example, the solution containing the (R)-amine can be obtained by contacting a mixture comprising the (S)-amine and the (R)-amine, e.g. racemic amine, with an optically active acid in a solvent or solvent mixture to give a mixture of diastereomeric acid addition salts and separating the resulting mixture to obtain the (S)-amine acid addition salt, e.g. by filtration of the crystallized amine acid addition salt, and an (R)-amine acid addition salt enriched mother liquor which is converted to a solution containing the free (R)-amine. In such a case the optically active acid can be selected from the group consisting of (-)-camphorsulfonic acid, (-)-dibenzoyl tartaric acid, (+)-dibenzoyl tartaric acid, N-formyl-L-aspartic acid, N-formyl-D-aspartic acid, N-acetyl-L-aspartic acid, N-formyl-L-glutamic acid and N-acetyl-L-glutamic acid. Preferably, N-acetyl-L-glutamic and most preferably (+)-dibenzoyl tartaric acid is used.

In a particularly preferred embodiment, the starting solution containing the (R)-amine is recovered from the mother liquor obtained in the separation step of step b) of the process according to the first aspect of the invention.

Generally, in step a) any suitable racemization agent can be used. Preferably, step a) of the process of the third aspect of the invention is performed as described above for to the process of the second aspect of the invention.

Furthermore, in a particularly preferred embodiment steps b), c) and d) of the process according to the third aspect of the invention are carried out as described above for steps a) to c) of the process of the first aspect of the invention.

Optionally, after step d) steps a) to d) are repeated once or several times.

In a fourth aspect, the invention relates to a process for the preparation of the racemic amine of formula II 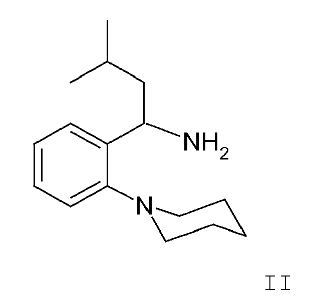 or a salt thereof,
comprising reacting the imine compound of formula VI 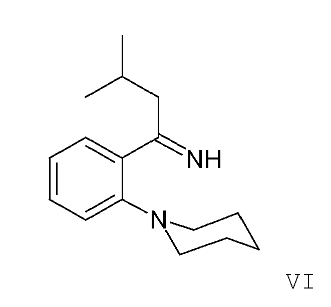 with a reducing agent, wherein the molar ratio of the imine compound to the reducing agent is less than 1:2.

Preferably, the molar ratio of the imine compound to the reducing agent is from about 1:1 to about 1:1.8 and particularly is from 1:1.2 to 1:1.5 such as about 1:1.3. Suitable examples for reducing agents include sodium or potassium borohydrides, LiAlH₄, sodium cyanoborohydrides, sodium and the like. Optionally, catalytical hydrogenation using any catalyst known in the art can be also applied. Preferably, NaBH₄ and more preferably NaBH₄ in methanolic solution is used as reducing agent.

Preferably, the reaction time is more than 3 hours such as about 4 hours. In particular, the reaction is performed at about 1 hour at about 0°C to 5°C, then about 1 hour at about 15°C and then about 2 hours at room temperature.

In a particularly preferred embodiment, the chemical reduction process is performed by slowly adding methanol to a cooled mixture comprising the imine compound and stirring this mixture for another hour at about 0°C to 5°C. Then, as a preferred reducing agent, NaBH₄ is added in small portions over 1h. This mixture is preferably stirred for 1h at 15°C and further for 2h at room temperature, before THF is added and stirred over night for precipitation of the product. Purification of the precipitated product is preferably performed by filtration and washing with THF first, followed by rinsing with water twice.

If desired, the racemic amine can be converted into a salt by reacting with an inorganic acid such as HCl or an organic acid such as glutaric acid.

The imine compound of formula VI can be prepared by reacting 2-piperidino-benzonitrile of formula VII 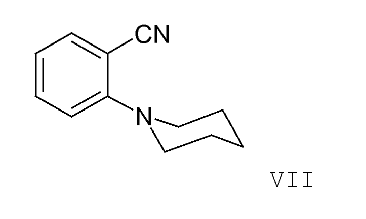 with isobutyl magnesium bromide (iBuMgBr).

The 2-piperidino-benzonitrile of formula VII can be prepared according to any process known in the art.

The molar ratio of 2-piperidino-benzonitrile to iBuMgBr is preferably less than 1:2.5 and more preferably is from 1:1 to 1:2.3 such as from 1:1.3 to 1:1.8 like about 1:1.5.

The reaction is preferably performed in toluene and the reaction temperature is preferably ranging from 100°C to 108°C and more preferably is from 103°C to 105°C.

In a preferred embodiment, 2-piperidino-benzonitrile is slowly added to a preheated mixture of toluene and iBuMgBr over 1h.

It is even more preferred to perform this reaction step at a temperature of about 87°C under nitrogen atmosphere. Further, the mixture is preferably heated to the reflux temperature of the solvent, and even more preferably to a temperature of about 103°C to 105°C for 3h.

If starting from 2-piperidino-benzonitrile of formula VII, the racemic amine of formula II is preferably prepared without isolating the imine compound of formula VI, i.e. in a one pot synthesis. Moreover, it is preferred that neither the imine compound of formula VI is purified by a basic extraction nor the racemic amine of formula II is purified by an acidic extraction.

The racemic amine of formula II prepared by a process according to the fourth aspect of the invention is suitably used as starting material for the process according to the first aspect of the invention.

In a fifth aspect, the invention relates to a process for the preparation of substantially optically pure repaglinide derivative of the formula III 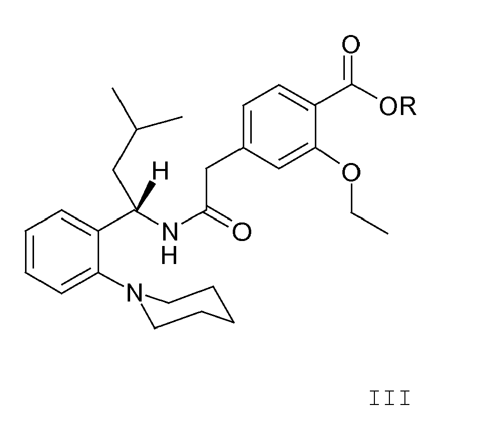 comprising reacting the substantially optically pure (S)-amine of formula I with a substituted acetic acid of the formula IV 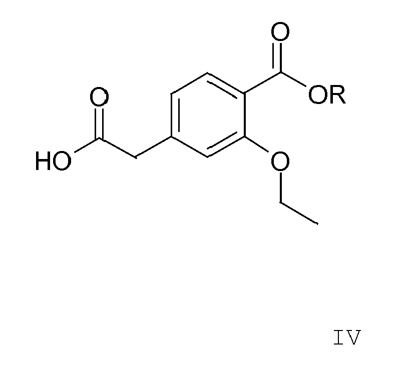 in a solvent and in the presence of an acid activating compound , wherein
a) R is selected from H or C₁-C₅ alkyl or R together with the benzoic acid moiety to which it is bound forms a salt;
   and
b) the acid activating compound is 4-(4,6-dimethoxy-1,3,5-triazin-2-yl)-4-methylmorpholinium chloride (DMT-MM);
   or
b) the acid activating compound and the solvent is selected from combinations of N,N'-carbonyldiimidazole in CH₂Cl₂; thionylchloride in toluene and/or triethylamine; or triphenylphosphine in CCl₄, triethylamine and/or acetonitrile.

In the present invention the substantially optically pure (S)-amine is preferably reacted with an ethyl ester derivative of the formula IV, wherein R is ethyl, to obtain substantially optically pure (S)(+)-2-ethoxy-4-[N-[1-(2-piperidinophenyl)-3-methyl-1-butyl]aminocarbonyl-methyl]-benzoic acid ethyl ester, which is also referred to as repaglinide ethyl ester.

In a preferred embodiment, the acid activating compound and the solvent are selected from combinations of N,N'-carbonyldiimidazole in CH₂Cl₂; thionylchloride in toluene and/or triethylamine; triphenylphosphine in CCl₄, triethylamine and/or acetonitrile; and 4-(4,6-dimethoxy-1,3,5-triazin-2-yl)-4-methylmorpholinium chloride (DMT-MM) in THF.

Thus, if the acid activating compound is N,N'-carbonyldiimidazole, then the solvent preferably is CH₂Cl₂. If the acid activating compound is thionylchloride, then the solvent preferably is toluene and/or triethylamine, most preferably a mixture having a volume ratio of toluene to triethylamine from 5:1 to 25:1 such as about 13:1. If the acid activating compound is triphenylphosphine, then the solvent preferably is CCl₄, triethylamine and/or acetonitrile, most preferably a mixture having a volume ratio of CCl₄ to triethylamine to acetonitrile of about 1:4.5:27. If the acid activating compound is DMT-MM, then the solvent preferably is THF.

In a particularly preferred embodiment, the substantially optically pure (S)-amine used for the preparation of substantially optically pure repaglinide derivative of the formula III according to the fifth aspect of the invention has been prepared as described above in a process according to the first or third aspect of the invention, preferably by a process according to the first aspect of the invention. Thus, optically pure (S)-amine prepared according to the present invention can be used for coupling with the substituted acetic acid of formula IV to give substantially optically pure repaglinide derivative of the formula III.

The substituted acetic acid of formula IV can be prepared by any process known in the art (e.g. J. Med. Chem. 1998, 41, 5219-5246 or WO 01/35900).

It has surprisingly been found that selecting the above mentioned activating compounds or combinations of acid activating compounds and solvents in the amide coupling reaction substantially improves the yield and purity of the repaglinide derivative of formula III, such as repaglinide ethyl ester, compared to the prior art. A further advantage relative to the processes disclosed in the prior art is that no further purification by column chromatography is necessary to obtain a high yield and purity, which renders the process more economical.

The invention is also directed to the use of substantially optically pure (S)-amine for the preparation of substantially optically pure repaglinide or a pharmaceutically acceptable salt, solvate or ester thereof, wherein the substantially optically pure (S)-amine has been prepared by a process according to the first aspect of the invention or according to a process according to the third aspect of the invention. Moreover, the substantially optically pure (S)-amine prepared by a process according to the first aspect of the invention or according to a process according to the third aspect of the invention can be used as a resolving agent for resolution of racemic Repaglinide to give substantially optically pure (S)-Repaglinide or a pharmaceutically acceptable salt, solvate or ester thereof.

Furthermore, the invention is also directed to the use of racemic amine of formula II for the preparation of substantially optically pure Repaglinide or a pharmaceutically acceptable salt, solvate or ester thereof, wherein the racemic amine has been prepared by a process according to the fourth aspect of the invention.

In addition, the invention is also directed to the use of substantially optically pure Repaglinide derivative of the formula III for the preparation of substantially optically pure repaglinide or a pharmaceutically acceptable salt, solvate or ester thereof, wherein the substantially optically pure repaglinide derivative of the formula III has been prepared by a process according to the fifth aspect of the invention.

Finally, the invention relates to a process for the preparation of substantially optically pure Repaglinide of the formula V 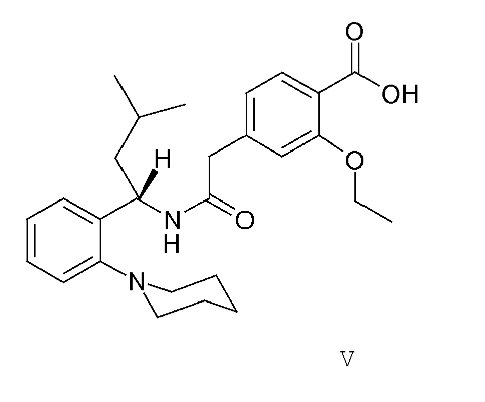 or a pharmaceutically acceptable salt or solvate thereof, which includes at least one step of:
i) preparing the substantially optically pure (S)-amine of the formula I by a process according to the first or third aspect of the invention, preferably by a process according to the first aspect of the invention; or
ii) preparing the racemic amine of the formula II by a process according to the fourth aspect of the invention; or
iii) preparing the substantially optically pure repaglinide derivative of the formula III by a process according to the fifth aspect of the invention; or
which includes each of the steps (i) to (iii).

In a particularly preferred embodiment, the process for the preparation of substantially optically pure Repaglinide comprises the steps (i) and (iii). The combination of these preparation steps is highly efficient with respect to the yield and purity of the repaglinide product.

Substantially optically pure Repaglinide prepared according to the present invention can also be used in pharmaceutical compositions that exhibit hypoglycemic effect and are used in long-term antidiabetic treatment which have been previously described in e.g. EP 0 147 850, EP 0 207 331, EP 0 208 200, EP 0 589 874, US 6830759 and IPCOM000142425D.

The present invention will further be illustrated by the following examples:

### Examples

### Example 1 (S)(+)-1-(2-piperidino-phenyl)-3-methyl-1-butylamine O,O-dibenzoyl-(+)-tartrate

A solution of 0.512 g of racemic 1-(2-piperidino-phenyl)-3-methyl-1-butylamine in 1.7 mL of butyl acetate was combined with of 0.372 g of O,O-dibenzoyl-(+)-tartaric acid at 60°C. The mixture was shortly heated to reflux and stirred until a clear solution was obtained. The mixture was then allowed to cool down to 45°C, seed crystals of (S)(+)-1-(2-piperidino-phenyl)-3-methyl-1-butylamine O,O-dibenzoyl-(+)-tartrate were added and the instantly precipitating salt was allowed to crystallize without disturbance for 12 hours at -10°C. Thereafter, the clear supernatant was carefully separated from the crystal cake of (S) (+)-1-(2-piperidino-phenyl)-3-methyl-1-butylamine O,O-dibenzoyl-(+)-tartrate salt by filtration. The solid salt was washed thoroughly with cold butyl acetate twice (2 x 0.3 ml). (S)(+)-1-(2-piperidino-phenyl)-3-methyl-1-butylamine 0,0-dibenzoyl-(+)-tartrate was obtained in an optical purity of 99.8% (e.e., determined by chiral HPLC; m.p. 142-144°C). For conversion to the corresponding free base, the salt was treated with ammonia/toluene.

Chiral HPLC analysis was performed under the following conditions:
- Column:: Gemini C6-Phenyl, 3 µm, 100 x 4.6 mm;
- Mobile phase:: solvent A: 0.01 M diammonium hydrogen phosphate pH 6.0;
solvent B: acetonitrile : methanol = 70 : 30
- Gradient::

| Time (min) | % A | % B |
|---|---|---|
| 0 | 70 | 30 |
| 2 | 70 | 30 |
| 3 | 63 | 37 |
| 18 | 63 | 37 |
| 28 | 10 | 90 |
| 31 | 10 | 90 |
| 33 | 70 | 30 |

| | |
|---|---|
| Post run: | 2 minutes |
| Column temp.: | 20 °C |
| Flow: | 1 ml per min |
| Detection: | UV, wavelength 210 nm |
| Injection: | 5 µl |

### Solvents and reagents:

1. water: purified, for chromatography;
2. acetonitrile, CH₃CN, liquid chromatography grade;
3. diammonium hydrogen phosphate, liquid chromatography grade;
4. 85% phosphoric acid solution;
5. solvent for dissolution of samples: 80% acetonitrile pH = 7.5;
6. solvent A: 0.02 M diammonium hydrogen phosphate pH 6.0: Accurately weight about 2.64 g of diammonium hydrogen phosphate and dissolve in about 950 ml of water. Adjust with phosphoric acid solution to a pH 6.0, transfer into a 1000 ml volumetric flask and dilute with water to volume. Filter through a 0.22 µm membrane filter and degas.
7. solvent B: acetonitrile : MeOH = 70 : 30

- Sample solution (SaS):: about 1.5 mg of the sample per ml.

### Example 2 (S) (+)-1-(2-piperidino-phenyl)-3-methyl-1-butylamine O,O-dibenzoyl-(+)-tartrate

0.216 g of O,O-dibenzoyl-(+)-tartaric acid were added to a solution of 0.3 g racemic 1-(2-piperidino-phenyl)-3-methyl-1-butylamine in a mixture of 1 ml isobutylmethyl ketone and 0.1 ml 1-butanole at 85°C. This mixture was stirred until a clear solution was obtained. Seeding crystals of (S)(+)-1-(2-piperidino-phenyl)-3-methyl-1-butylamine O,O-dibenzoyl-(+)-tartrate were added at a temperature of 65°C, whereupon crystallization occurred after 10-15 minutes. The thus obtained suspension was stirred for 8h at -15°C. After filtration, the precipitate was washed on the filter three times with 0.3 ml of isobutylmethyl ketone at 5°C and dried under an infrared lamp. (S)(+)-1-(2-piperidino-phenyl)-3-methyl-1-butylamine O,O-dibenzoyl-(+)-tartrate was obtained in an optical purity of 100% (e.e.), as determined by chiral HPLC.

### Example 3 (S)(+)-1-(2-piperidino-phenyl)-3-methyl-1-butylamine O,O-dibenzoyl-(+)-tartrate

0.216 g of O,O-dibenzoyl-(+)-tartaric acid was added to a solution of 0.3 g racemic 1-(2-piperidino-phenyl)-3-methyl-1-butylamine in 1 ml of isobutyl methyl ketone at 85°C. This mixture was stirred until a clear solution was obtained, which was then allowed to crystallize without disturbance for 48 hours at -15°C. After filtration, the precipitate was washed on the filter three times with 0.3 ml of isobutyl methyl ketone at 5°C and dried under an infrared lamp. (S)(+)-1-(2-piperidino-phenyl)-3-methyl-1-butylamine O,O-dibenzoyl-(+)-tartrate was obtained in an optical purity of 100% (e.e.), determined by chiral HPLC.

### Examples 4-8 (S)(+)-1-(2-piperidino-phenyl)-3-methyl-1-butylamine O,O-dibenzoyl-(+)-tartrate

Example 3 was repeated with the exception that instead of isobutyl methyl ketone a solvent or solvent mixture as indicated in the table below was used.

| Example | Solvent | Yield (%) | optical purity (% (S), HPLC) |
|---|---|---|---|
| 4 | 2-pentanone | 19 | 93 |
| 5 | 2-butanone | 38 | 72 |
| 6 | isobutyl methyl ketone/toluene | 35 | 99 |
| 7 | butyl acetate/ 1-butanol | 32 | 100 |
| 8 | 2-methylpropyl acetate | 35 | 100 |

### Example 9 (-)-1-(2-piperidino-phenyl)-3-methyl-1-butylamine O,O-dibenzoyl-(-)-tartrate

A solution of 0.341 g of racemic 1-(2-piperidino-phenyl)-3-methyl-1-butylamine in 1 ml of butyl acetate was combined with 0.124 g of O,O-dibenzoyl-(-)-tartaric acid at 60°C. Then the mixture was shortly heated to reflux and stirred until a clear solution was obtained. After slow cooling to 0°C, the mixture was allowed to crystallize for 12 hours at -15°C without disturbance. Thereafter, the clear supernatant was carefully separated from the crystal cake of (-)-1-(2-piperidino-phenyl)-3-methyl-1-butylamine O,O-dibenzoyl-(-)-tartrate salt by filtration. The solid was washed thoroughly with refrigerated butyl acetate (0.3ml) and the washing was combined with the supernatant. (R) (-) -1- (2-piperidino-phenyl)-3-methyl-1-butylamine O,O-dibenzoyl-(-)-tartrate was obtained in a optical purity of 98.1% (e.e.), determined by chiral HPLC.

### Example 10 Racemization of (R)-enriched 1-(2-piperidino-phenyl)-3-methyl-1-butylamine dibenzoyl tartaric acid addition salt

The mother liquors of the first optical resolution of racemic 1-(2-piperidino-phenyl)-3-methyl-1-butylamine obtained in Examples 1 to 9 were evaporated to dryness to recover the (R)-enriched 1-(2-piperidino-phenyl)-3-methyl-1-butylamine dibenzoyl tartaric acid salt. The amine base was freed with ammonia and extracted with CH₂Cl₂. After complete evaporation of the solvent, the crude extract showed to contain 79.8% of the (R)-enantiomer (as determined by chiral HPLC), with a water content of 0.15%.

### Example 10a)

The regenerated (R)-enriched 1-(2-piperidino-phenyl)-3-methyl-1-butylamine was dried azeotropicaly with toluene. Its water content was controlled by Karl-Fischer-titration throughout the process (<0.05%). 30 mg of the thus obtained dry (R)-enriched 1-(2-piperidino-phenyl)-3-methyl-1-butylamine was dissolved in 1 ml of dry methanol. Then, 34 mg of potassium tert butoxide (t-BuOK) was added in dry box. This reaction mixture (carefully protected from moisture) was gently refluxed until a test portion showed an equalized S:R proportion, indicating the completeness of the racemization reaction. Thereafter, the solvent was evaporated in vacuo and the evaporation residue was redistributed in a mixture of 2.5 ml of water and 5 ml of CH₂Cl₂. The CH₂Cl₂ phase was separated, dried with anhydrous magnesium sulfate and evaporated in vacuo. The free 1-(2-piperidino-phenyl)-3-methyl-1-butylamine base was obtained in an (R):(S) ratio of 49:51 (as determined by chiral HPLC).

### Example 10b)

30 mg of the regenerated (R)-enriched 1-(2-piperidino-phenyl)-3-methyl-1-butylamine was dissolved in 3 ml of methanol and 20 mg of Lewatit CNP 105 was added. This reaction mixture was stirred at a temperature of about 25-30°C until a test portion showed an equalized S:R proportion, indicating the completeness of the racemization reaction. Thereafter, the reaction mixture was filtered, the filtrate cake was washed with methanol (2x1.5ml) and the solvent was evaporated in vacuo. Then, the evaporation residue was redistributed in a mixture of 2.5 ml water and 5 ml CH₂Cl₂, the CH₂Cl₂ layer was separated, dried with anhydrous magnesium sulfate and evaporated in vacuo. The free 1-(2-piperidino-phenyl)-3-methyl-1-butylamine base was obtained in an (R):(S) ratio of 53:47 (as determined by chiral HPLC).

### Example 10c)

20 mg of the regenerated (R)-enriched 1-(2-piperidino-phenyl)-3-methyl-1-butylamine was dissolved in 3 ml ethanol and 16 mg powdered potassium hydroxide was added in dry box. This reaction mixture was stirred for 12h at the reflux temperature of the solvent. Thereafter, the solvent was evaporated in vacuo and the evaporation residue was redistributed in a mixture of 5 ml of water and 5 ml of CH₂Cl₂. The CH₂Cl₂ phase was separated, dried with anhydrous magnesium sulfate and evaporated in vacuo. The dichloromethane layer was separated, successively washed with water, dried with anhydrous magnesium sulfate and evaporated in vacuo. The free 1-(2-piperidino-phenyl)-3-methyl-1-butylamine was obtained in an (R):(S) ratio of 64:36 (as determined by chiral HPLC).

### Example 11 (S)(+)-2-ethoxy-4-[N-[1-(2-piperidinophenyl)-3-methyl-1-butyl]aminocarbonyl-methyl]-benzoic acid ethyl ester

20.77 g (82.4 mmol) of 2-(3-Ethoxy-4-(ethoxycarbonyl)phenyl)-acetic acid were slowly added to a solution of N,N'-carbonyldiimidazole (13.36 g, 82.4 mmol, 97%) in dichloromethane (240 ml) at room temperature. After activation for 1.5h at 20-25°C a solution of (S)-3-methyl-1-(2-(piperidin-1-yl)phenyl)butan-1-amine (19.70 g, 80.0 mmol) in dichloromethane (80 ml) was added dropwise and the resulting mixture was stirred for 2h. The reaction mixture was quenched by adding water (2 x 160 ml). The organic phase was then evaporated under reduced pressure. The yield of the crude repaglinide ester product of formula IV was 35.7 g (93%; HPLC: 97.58%) .

### Example 12 (S)(+)-2-ethoxy-4-[N-[1-(2-piperidinophenyl)-3-methyl-1-butyl]aminocarbonyl-methyl]-benzoic acid ethyl ester

A solution of 2.65 g (10.5 mmol) of 2-(3-ethoxy-4-(ethoxycarbonyl)phenyl)acetic acid and 2 ml of thionylchloride was heated to reflux for 2-3h. The solvent was evaporated in vacuum and the residue (2.86 g, 10.56 mmol) was dissolved in toluene (20 ml) and triethylamine (1.5 ml, 10.6 mmol) was added at 20-25°C. Then to this reaction mixture a solution of (S)-1-(2-(piperidin-1-yl)phenyl)butan-1-amine (2.46 g, 10 mmol) in toluene (15 ml) was added dropwise at 20-25°C and maintained at this temperature for another 2h. The reaction was quenched by adding water (40 ml) and pH was adjusted to about 8-9 with 10% NaOH. After phase separation, the organic phase was again washed with water, dried with Na₂SO₄, filtrated and evaporated in vacuum to get 4.25 g (88.5%) of (S)(+)-2-ethoxy-4-[N-[1-(2-piperidinophenyl)-1-butyl]aminocarbonylmethyl]-benzoic acid ethyl ester.

### Example 13 (S)(+)-2-ethoxy-4-[N-[1-(2-piperidinophenyl)-3-methyl-1-butyl]aminocarbonyl-methyl]-benzoic acid ethyl ester

To a solution of (S)-1-(2-(piperidin-1-yl)phenyl)butan-1-amine (2.46 g, 10 mmol) in acetonitrile (25 ml), 2.52 g (10 mmol) of 2-(3-ethoxy-4-(ethoxycarbonyl)phenyl)acetic acid, triphenylphosphine (3.14 g, 12 mmol), triethylamine (4.2 ml, 30 mmol) and 0.94 ml (10 mmol) of tetrachloromethane were added. The reaction mixture was stirred at 20-25°C for 10h. The solvent was evaporated in vacuum and the residue was distributed in ethylacetate and water. The organic phase was dried, filtered and evaporated in vacuum. The crude residue was then purified by column chromatography (toluene/acetone=10/1) to give 4.37 g (91.0%) (S)(+)-2-ethoxy-4-[N-[1-(2-piperidinophenyl)-1-butyl]-aminocarbonyl-methyl]-benzoic acid ethyl ester.

### Example 14 (S)(+)-2-ethoxy-4-[N-[1-(2-piperidinophenyl)-3-methyl-1-butyl]aminocarbonyl-methyl]-benzoic acid ethyl ester

To a solution of (S)-1-(2-(piperidin-1-yl)phenyl)butan-1-amine (1.36 g, 5 mmol) and 1.26 g (5 mmol) of 2-(3-ethoxy-4-(ethoxycarbonyl)phenyl)acetic acid in THF (25 ml), 1.67 g (5.5 mmol) of 4-(4,6-dimethoxy-1,3,5-triazin-2-yl)-4-methylmorpholinium chloride (DMT-MM) was added at 20-25°C. After 3h the reaction was quenched by adding water and diethylether. The phases were separated and the aqueous phase was again washed with ether. The ethereal organic phases were dried, filtered and evaporated in vacuum to get the 2.2 g (91.7%) of crude (S)(+)-2-ethoxy-4-[N-[1-(2-piperidinophenyl)-1-butyl]aminocarbonyl-methyl]-benzoic acid ethyl ester.

### Comparative Example 1 (according to EP 0 147 850, Example 2) (S)(+)-2-ethoxy-4-[N-[1-(2-piperidinophenyl)-1-butyl]aminocarbonyl-methyl]-benzoic acid ethyl ester

0.90 g (3.57 mmol) 2-(3-Ethoxy-4-(ethoxycarbonyl)phenyl)acetic acid together with 0.61 g (3.73 mmol) N,N'-carbonyldiimidazole in 9 ml THF were heated to reflux for 5h. Thereafter, a solution of 0.85 g (3.67 mmol) (S)-1-(2-(piperidin-1-yl)phenyl)butan-1-amine (e.e. = 94.2) in 9 ml THF was added and heated to reflux for 3h. The solvent was evaporated in vacuo and the residue was distributed in chloroform and water. The organic phase was dried, filtered and evaporated in vacuo. The crude residue was then purified by column chromatography (SiO₂) to give a yield of 0.85 g (51.2%).

### Example 15 racemic 1-(2-piperidino-phenyl)-3-methyl-1-butylamine glutaric acid salt

A mixture of iBuMgBr (2 M in diethyl ether, 1.6 l, 3.2 mol) and toluene (1.6 1) was heated to a temperature of about 87°C under nitrogen atmosphere. Then a solution of 2-piperidino-benzonitril (300 g, 1.6 mol) in toluene (1.6 1) was slowly added to the hot mixture within 1h, while maintaining the temperature. Thereafter, the reaction mixture was heated to reflux at a temperature of about 103-105°C for 3h. After cooling the mixture to 0°C, 1.6 1 of methanol were slowly added to this mixture. The resulting mixture was stirred for another hour at about 0-5°C. Then NaBH₄ (121 g, 3.2 mol) was added successively within 1h. Thereafter, the mixture was stirred for another hour at 15°C and for another 2 hours at room temperature. Then 1.6 1 of THF was added and the mixture was stirred over night. The precipitated salts were filtrated and washed with THF (2 x 400 ml) and water (2 x 1.6 1) twice. The filtrate was dried and evaporated in vacuo to give 352 g of a crude yellow oil, which was then dissolved in 2-propanole (3.2 1). This solution was heated to a temperature of about 50°C, at which 180 g of glutaric acid was slowly added. The product precipitated from this solution was stirred for 2h at room temperature and for 2h at 0-5°C. After filtration and washing with cold 2-propanole (300 ml), the wet product was dried in a vacuum drier at 60°C for 18h, to give 463 g (76%) of the desired product.

### Example 16 racemic 1-(2-piperidino-phenyl)-3-methyl-1-butylamine glutaric acid salt

A mixture of iBuMgBr (2M in ether, 15 ml, 30 mmol) and toluene (15 ml) was heated to a temperature of about 87°C under nitrogen atmosphere. Then a solution of 2-piperidino-benzonitril (4.45 g, 23 mmol) in toluene (15 ml) was slowly added to the hot mixture within 1h, while maintaining the temperature. Thereafter, the reaction mixture was heated to reflux at a temperature of about 103-105°C for 3h. After cooling the mixture to 0°C, 15 ml of methanol was slowly added. This mixture was stirred for another hour at about 0-5°C. Then NaBH₄ (1.32 g, 34.5 mmol) was added successively within 1h. Thereafter, the mixture was stirred for another hour at 15°C and for another 2 hours at room temperature. Then 15 ml of THF was added and the mixture was stirred over night. The precipitated salts were filtrated and washed with THF (2 x 400 ml) and water (2 x 1.6 l) twice. The filtrate was dried and evaporated in vacuo to give 4.98 g (87%) of crude yellow oil, which was then dissolved in 2-propanole (50 ml). This solution was heated to a temperature of about 50°C, at which glutaric acid (2.64 g, 20 mmol) was slowly added. The product precipitated from this solution was stirred for 2h at room temperature and for 2h at 0-5°C. After filtration and washing with cold 2-propanole (10 ml), the wet product was dried in a vacuum drier at 60°C for 18h, to give 6.05 g (70%) of the desired product.

### Comparative Example 2 (according to J. Med. Chem. 1998,vol. 41,p 5234-5235) racemic 1-(2-piperidino-phenyl)-3-methyl-1-butylamine glutaric acid salt

To a solution of i-BuMgBr (268 mmol) in 135 mL of toluene/THF (4:1) was added a solution of 2-piperidino-benzonitril (107 mmol) in 135 mL of toluene/THF (4:1). After reflux for 3 h and standing overnight, the reaction mixture was dropped into a cold (-15 °C) mixture of concentrated ammonia (300 ml) and saturated aqueous NH₄Cl (300 ml). After the mixture was stirred for 10 min, the mixture was filtered through a layer of silica gel, and the organic layer was separated, dried, and evaporated in vacuum to give the (3-Methyl-butyl)-(2-piperidino-phenyl)-ketimine as yellow oil (crude, 100%). NaBH₄ (161 mmol) was added within 1 h to crude (3-Methyl-butyl)-(2-piperidino-phenyl)-ketimine (80.5 mmol) in MeOH (160 mL) at 0 °C; stirring was continued at 15 °C for 2 h. The reaction was evaporated in vacuum, and 10% aqueous HCl (100 mL) was added cautiously under cooling with ice. After extraction with CH₂Cl₂ (4 x40 mL, discarded), the aqueous phase was alkalinized with 50% NaOH and extracted with CH₂Cl₂ (2 X 50 mL). The organic layer was washed with water (40 mL), dried, and evaporated in vacuum. The residue was dissolved in EtOH (25 mL) and cooled in ice. A solid product was filtered off, and the filtrate was evaporated in vacuo to give the desired product as yellow oil (65%) which was used for further reaction. For purification and/or storage, the glutaric acid salt (m.p. 179-180°C) was formed.

### Example 17 (S)(+)-1-(2-piperidino-phenyl)-3-methyl-1-butylamine O,O-dibenzoyl-(+)-tartrate

50 g of racemic 1-(2-piperidino-phenyl)-3-methyl-1-butylamine glutaric acid salt were suspended in dichloromethane (500 mL) and water (250 mL). 4M NaOH was added for neutralization and the pH was adjusted to 8 to 9. After separation of phases the organic phase was washed with water (100mL), dried with anhydrous Na₂SO₄, filtrated and evaporated under reduced pressure to give 31,2 g (96%) of racemic 1-(2-piperidino-phenyl)-3-methyl-1-butylamine as yellow oil. This intermediate product was then converted to (S)(+)-1-(2-piperidino-phenyl)-3-methyl-1-butylamine O,O-dibenzoyl-(+)-tartrate according to a process as described in Examples 1 to 3.

### Example 18 (S)(+)-1-(2-piperidino-phenyl)-3-methyl-1-butylamine

Example 15 was repeated to give racemic 1-(2-piperidino-phenyl)-3-methyl-1-butylamine which was then converted to substantially optically pure (S) (+)-1-(2-piperidino-phenyl)-3-methyl-1-butylamine as described in Example 1.

### Example 19 (S)(+)-2-ethoxy-4-[N-[1-(2-piperidinophenyl)-3-methyl-1-butyl]aminocarbonyl-methyl]-benzoic acid (Repaglinide)

To a hot solution (40-45°C) of crude (S)(+)-2-ethoxy-4-[N-[1-(2-piperidinophenyl)-3-methyl-1-butyl]aminocarbonyl-methyl]-benzoic acid ethyl ester (35.7 g) in ethanol (320 ml), 112 ml of 1M NaOH (112 mmol) was added dropwise. After heating this mixture to a temperature of about 55-58°C for 2.5h, charcoal was added for 15 minutes. Then, the hot suspension was filtrated. The filtrate was cooled to about 35°C and 112 ml of 1M HCl (112mmol) was added slowly to give a suspension having a pH of about 5. After keeping this mixture at a temperature of about 20-25°C for 2h and for another 2h at about 0-5°C, the precipitated product was filtrated and washed with 30 ml of cooled ethanol 50%. The crude product was crystallized from a 2:1 mixture of EtOH and water (450 ml) and dried at 50°C for 5h to obtain the desired (S)-repaglinide in a yield of 72% (28.5 g).

## Claims

1. A process for the preparation of substantially optically pure (S)-amine of the formula I 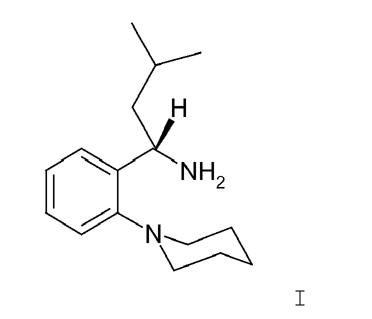 comprising:
a) contacting racemic amine of the formula II 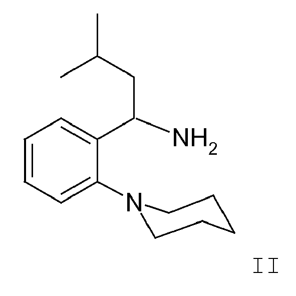 with an optically active acid in a solvent or solvent mixture to give a mixture of diastereomeric acid addition salts, wherein the optically active acid is (+)-dibenzoyl tartaric acid,
b) separating the mixture of diasteromeric acid addition salts to obtain substantially optically pure (S)-amine acid addition salt, and
c) converting the (S)-amine acid addition salt obtained in step b) to substantially optically pure (S)-amine of formula I.

2. The process according to claim 1, wherein the (S)-amine of formula I has an optical purity of at least 70% (e.e.), preferably at least 90 % (e.e.), more preferably at least 95% and most preferably 100% (e.e).

3. The process according to claim 1 or 2, wherein the solvent is selected from the group consisting of isobutylmethyl ketone, 1-butanole, methylethyl ketone, butyl acetate, 2-methylpropyl acetate, 2-butanone, 2-pentanone, toluene, 2-methylethyl acetate and mixtures thereof.

4. The process according to any of claims 1 to 3, wherein in step a) the optically active acid is used in an amount of from 0.25 to 0.75 molar equivalents and preferably in an amount of about 0.5 molar equivalents, with respect to the racemic amine of the formula II.

5. The process according to any of claims 1 to 4, wherein step a) is performed at a temperature of about 50°C to the reflux temperature of the solvent, and preferably at a temperature of 60°C to 90°C.

6. The process according to any of claims 1 to 5, wherein step b) is performed at a temperature of about -20°C to about 20°C, preferably at a temperature of about -20°C to 10 °C, and even more preferably at a temperature of about -20°C to -10°C.

7. The process according to any of claims 1 to 6, wherein prior to step b) the mixture of diasteromeric acid addition salts is seeded with crystals of (S)(+)-1-(2-piperidino-phenyl)-3-methyl-1-butylamine O,O'-dibenzoyl-(+)-tartrate, preferably at a temperature of about 0°C to the boiling point of the solvent or solvent mixture, more preferably at a temperature of about 30°C to about 60°C, and most preferably at a temperature of about 40°C to about 50°C.

8. A process for the preparation of a mixture comprising the (S)-amine of formula I 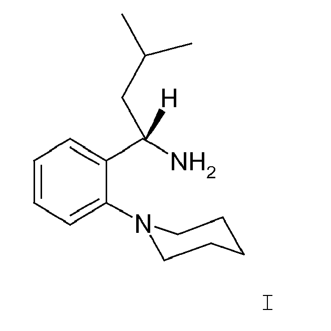 and the (R)-amine of formula VIII 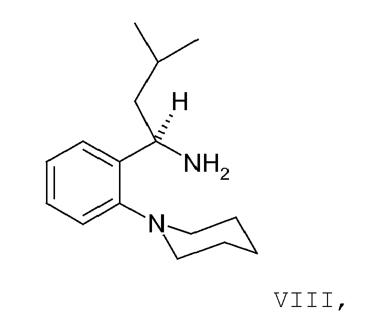 comprising treating a solution containing the (R)-amine with a racemization agent selected from the group consisting of ion exchange resins, potassium tert.-butoxide (t-BuOK) and mixtures thereof, wherein the amount of the (S)-amine in the obtained mixture is higher than in the solution containing the (R)-amine.

9. A process for the preparation of substantially optically pure (S)-amine of the formula I 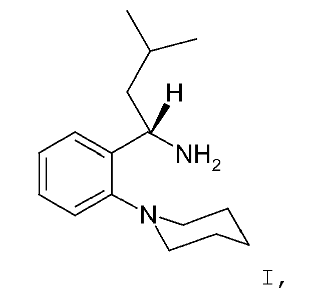 comprising:
a) treating a solution containing the (R)-amine of formula VIII 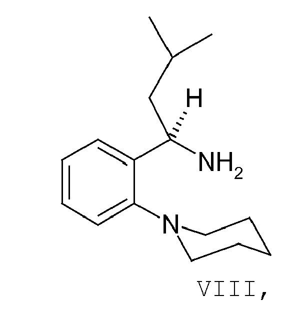 with a racemization agent to obtain a mixture comprising the (S)-amine of formula I and the (R)-amine of formula VIII, wherein the amount of the (S)-amine in the obtained mixture is higher than in the solution containing the (R)-amine;
b) contacting the mixture obtained in step a) with O,O'-dibenzoyl-(+)-tartaric acid in a solvent or solvent mixture to give a mixture of diastereomeric acid addition salts;
c) separating the mixture of diastereomeric acid addition salts to obtain substantially optically pure (S)-amine acid addition salt and
d) converting the substantially optically pure (S)-amine acid addition salt obtained in step c) to substantially optically pure (S)-amine of formula I.

10. A process for the preparation of the racemic amine of formula II 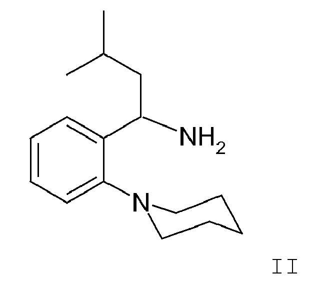 comprising reacting the imine compound of formula VI 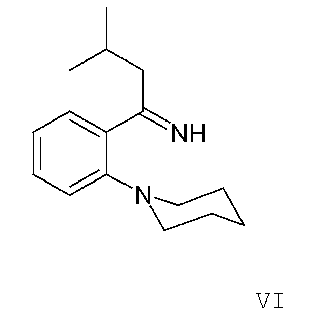 with a reducing agent, wherein the molar ratio of the imine compound to the reducing agent is less than 1:2.

11. Process according to claim 10, wherein the imine compound of formula VI is prepared by reacting 2-piperidino-benzonitrile of formula VII 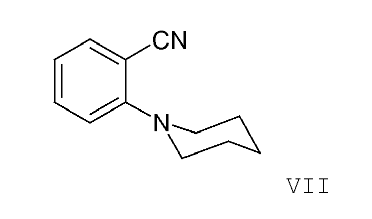 with isobutyl magnesium bromide (iBuMgBr).

12. The process according to claim 1, wherein the racemic amine of formula II has been prepared by a process according to claim 10 or 11.

13. Use of substantially optically pure (S)-amine of formula I for the preparation of substantially optically pure repaglinide or a pharmaceutically acceptable salt, solvate or ester thereof, wherein the substantially optically pure (S)-amine of formula I has been prepared by a process according to any of claims 1 to 7 or 9 or 12.

14. A process for the preparation of substantially optically pure Repaglinide derivative of the formula III 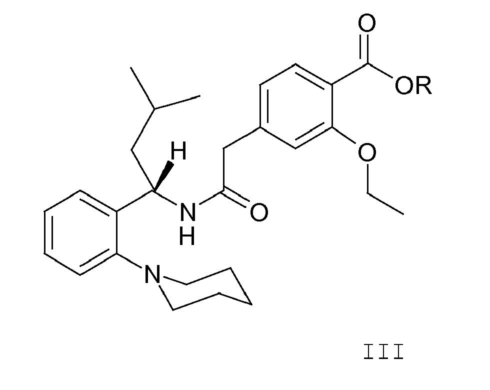 comprising reacting the substantially optically pure (S)-amine of formula I with a substituted acetic acid of the formula IV 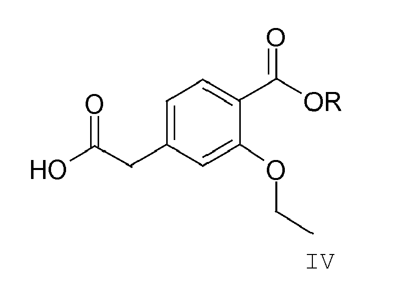 in a solvent and in the presence of an acid activating compound, wherein
a) R is selected from H or C₁-C₅ alkyl or R together with the benzoic acid moiety to which it is bound forms a salt;
and
b) the acid activating compound is 4-(4,6-dimethoxy-1,3,5-triazin-2-yl)-4-methylmorpholinium chloride (DMT-MM);
or
b) the acid activating compound and the solvent is selected from combinations of N,N'-carbonyldiimidazole in CH₂Cl₂; thionylchloride in toluene and/or triethylamine; or triphenylphosphine in CCl₄, triethylamine and/or acetonitrile.

15. Process according to claim 14, wherein R is ethyl.

16. Process according to any of claims 14 or 15, wherein the substantially optically pure (S)-amine of formula I has been prepared by a process according to any one of claims 1 to 7 or 9 or 12, preferably by a process according to any one of claims 1 to 7 or 12.

17. A process for the preparation of substantially optically pure Repaglinide of the formula V 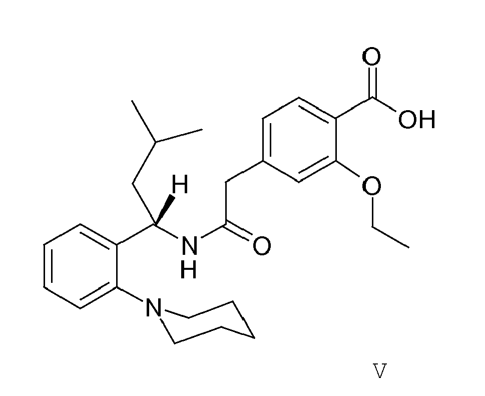 or a pharmaceutically acceptable salt or solvate thereof, which includes at least one step of:
i) preparing the substantially optically pure (S)-amine of the formula I according to the process of any one of claims 1 to 7 or 9 or 12; or
ii) preparing the racemic amine of formula II according to the process of claim 10 or 11; or
iii) preparing the substantially optically pure repaglinide derivative of the formula III according to the process of any one of claims 14 to 16; or
which includes each of the steps (i) to (iii).
